(19) Europäisches Patentamt
European Patent Office
Office européen des brevets

(11) EP 3 031 892 B1

(12) EUROPEAN PATENT SPECIFICATION

(45) Date of publication and mention of the grant of the patent: **26.02.2020 Bulletin 2020/09**

(21) Application number: **15199329.2**

(22) Date of filing: **10.12.2015**

(51) Int Cl.:
*C11D 3/37* (2006.01) *C11D 3/20* (2006.01)
*C11D 1/22* (2006.01) *C11D 1/29* (2006.01)
*A61K 8/34* (2006.01) *A61K 8/36* (2006.01)
*A61K 8/58* (2006.01) *A61Q 5/12* (2006.01)
*A61K 8/89* (2006.01) *A61Q 19/00* (2006.01)
*C11D 17/00* (2006.01) *C11D 11/00* (2006.01)
*C11D 3/00* (2006.01) *A61K 8/04* (2006.01)

(54) **A PROCESS FOR MAKING A CONSUMER GOODS PRODUCT COMPRISING A BENEFIT DELIVERY COMPOSITION**

VERFAHREN ZUR HERSTELLUNG EINEM GEBRAUCHSGUT ENTHALTEND EINE VORTEILSAUSGABEZUSAMMENSETZUNG

PROCÉDÉ DE FABRICATION D'UN BIEN DE CONSOMMATION CONTENANT UNE COMPOSITION D'ADMINISTRATION DE BÉNÉFICE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **12.12.2014 EP 14197591**

(43) Date of publication of application: **15.06.2016 Bulletin 2016/24**

(73) Proprietor: **The Procter & Gamble Company**
**Cincinnati, OH 45202 (US)**

(72) Inventors:
- **MASSEY-BROOKER, Anju Deepali**
  **Newcastle upon Tyne, NE12 9TS (GB)**
- **VACCARO, Mauro**
  **Newcastle upon Tyne, NE12 9TS (GB)**
- **ROBLES, Eric San Jose**
  **Newcastle upon Tyne, NE12 9TS (GB)**
- **SOMERVILLE ROBERTS, Nigel Patrick**
  **Newcastle upon Tyne, NE12 9TS (GB)**
- **CUTHBERTSON, Melissa**
  **Newcastle upon Tyne, NE12 9TS (GB)**
- **HATZOPOULOS, Marios**
  **Newcastle upon Tyne, NE12 9TS (GB)**
- **MCMEEKIN, Yvonne Bridget**
  **Newcastle upon Tyne, NE12 9TS (GB)**
- **CLARE, Jonathan Richard**
  **Newcastle upon Tyne, NE12 9TS (GB)**

(74) Representative: **P&G Patent Belgium UK**
**N.V. Procter & Gamble Services Company S.A.**
**Temselaan 100**
**1853 Strombeek-Bever (BE)**

(56) References cited:
**WO-A2-96/02229** **WO-A2-2009/072027**
**US-A1- 2003 223 952** **US-A1- 2006 024 256**
**US-A1- 2012 093 757**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

### FIELD OF THE INVENTION

The present invention invention relates to a process for making a benefit delivery composition. The process provides a benefit delivery composition having excellent silicone deposition, and excellent product stability profiles.

### BACKGROUND OF THE INVENTION

Silicones are incorporated into a variety of compositions, such as consumer goods products. In some applications, such as laundry treatment, hair treatment, and skin treatment, it is desirable for these silicones to be delivered onto the surface to be treated during the treatment process. However, silicones are typically incorporated into these consumer goods products at very low levels, and the efficiency of silicone deposition onto the treated surface during the treatment process is also low. There remains a need to improve the deposition of silicone onto a treated surface during the treatment process. A benefit delivery composition comprising silicone, surfactant and a fatty amphiphile can be used to deposit the silicone onto a treated surface during a treatment process.

The Inventors have found that the stability of a resultant benefit delivery composition is significantly improved when a benefit delivery composition is prepared by a process that (i) forms a lamellar phase from a surfactant and fatty amphiphile under specific conditions. Such a process provides a benefit delivery composition that exhibits excellent silicone deposition, and excellent product stability.

Such benefit delivery compositions can be incorporated into consumer goods products such as fabric treatment compositions (including laundry detergent compositions), hair treatment compositions (including shampoos and conditioners) and skin treatment compositions (including moisturizer creams and shaving creams). US2006/024256 relates to process for preparing a shampoo composition, said process comprising the steps of: a) combining a fatty amphiphile, a secondary surfactant, and water at a temperature sufficient to allow partitioning of said secondary surfactant and said water into said fatty amphiphile to form a pre-mix; b) cooling said pre-mix below the chain melt temperature of said fatty amphiphile to form a gel network; c) adding said gel network to one or more detersive surfactants and an aqueous carrier to form a shampoo composition.

US2012/0093757 relates to a hair conditioner comprising silicone and a conditioning gel phase.

US2003/0223952 relates to a shampoo comprising detersive surfactant, fatty alcohol gel network and an aqueous carrier.

WO2003/15736 relates to an aqueous composition comprising surfactant, silicone and perfume. In the examples of this reference, the composition is formed by mixing the ingredients together (c.f. example 1). No pre-mixes were formed, and the surfactant was not contacted to a fatty amphiphile at the elevated temperature required to form a lamellar phase composition.

WO2004/24114 relates to a composition comprising a fragrance composition and a silicone in water emulsion comprising at least one surfactant. In the examples of this reference, the silicone is contacted with the surfactant to form the emulsion, and then perfume is mixed with the emulsion. The surfactant was not contacted to a fatty amphiphile at the elevated temperature required to form a lamellar phase composition.

WO2006/012767 relates to a fluid personal care product comprising a fragrance that is dissolved in a silicone oil. In this reference, a silicone/perfume premix is formed, which can then be contacted to a surfactant, e.g. to form an emulsion or to contact with a surfactant phase prior to blending the premix with the other ingredients to give the fluid product (c.f. page 4, lines 12-18). In this reference, the silicone was not contacted with a lamellar phase composition. In this reference, no lamellar phase composition was formed: the surfactant was not contacted to a fatty amphiphile at the elevated temperature required to form a lamellar phase composition.

WO2008/110590 relates to a liquid surfactant composition comprising a cleansing surfactant, polymeric cationic material and an anionic or non-ionic emulsion of a fragrance composition blended with a waxy silicone material. In this reference, a perfume is blended with a silicone. In paragraph 0022, this blend can be formed by emulsification using a surfactant. In the examples (c.f. paragraph 0034), the silicone was contacted with the surfactant, and then perfume was added to this mixture. In this reference, no lamellar phase composition was formed: the surfactant was not contacted to a fatty amphiphile at the elevated temperature required to form a lamellar phase composition.

### SUMMARY OF THE INVENTION

A process for making a consumer goods product comprising a benefit delivery composition, wherein the process comprises the steps of:

(a) contacting a surfactant and a fatty amphiphile to form a lamellar phase composition;

(b) contacting the lamellar phase composition with silicone to form the benefit delivery composition,
(c) contacting the benefit delivery composition with at least three different consumer goods products ingredients to form a consumer goods product,

wherein the fatty amphiphile has a melting point of at least 40°C, wherein in step (a) the fatty amphiphile is at a temperature above its melting point when it is contacted with the surfactant,
wherein the fatty amphiphile within the lamellar phase composition is subsequently cooled to a temperature below the melting point of the fatty amphiphile, wherein the benefit delivery composition comprises from 50wt% to less than 90wt% lamellar phase composition and from greater than 10wt% to 50wt% silicone,
and wherein the fatty amphiphile is selected from fatty acid, fatty alcohol and mixtures thereof,
wherein step (b) is carried out under conditions of low shear, having a maximum tip speed of $2.5ms^{-1}$, preferably $2.0ms^{-1}$, or even $1.5ms^{-1}$, and at maximum shear rate of $500s^{-1}$

DETAILED DESCRIPTION OF THE INVENTION

**Benefit delivery composition:** The benefit delivery composition comprises silicone and a lamellar phase composition. The benefit delivery composition tconsists essentially only of the lamellar phase composition and silicone. The silicone and lamellar phase composition are described in more detail below. The benefit delivery compositon comprises silicone, surfactant and fatty amphiphile: these ingredients are described in more detail below. The benefit delivery composition preferably comprises water.Preferably, the benefit delivery composition comprises from 50wt% to less than 90wt% lamellar phase compositon, more preferably from 60wt% to 80wt% lamellar phase composition. Preferably, the benefit delivery composition comprises from greater than 10wt% to 50wt% silicone, more preferably from 20wt% to 40wt% silione. Preferably, the weight ratio of lamellar phase composition to silicone present in the benefit delivery agent is in the range of from 1:1 to 8:1, preferably from 2:1 to 6:1, or from 2:1 to 4:1.

Preferably, the benefit delivery composition comprises from greater than 10wt% to 48wt% silicone, more preferably from 20wt% to 35wt% silicone.

Preferably, the benefit delivery composition comprises from 10wt% to 30wt% surfactant, more preferably from 10wt% to 20wt% surfactant. Preferably, the benefit delivery composition comprises from 5wt% to 20wt% fatty amphiphile, more preferably from 5wt% to 10wt% fatty amphiphile. The benefit delivery composition may also comprise water, preferably from 0wt% water to 75wt% water, more preferably 20wt% to 75wt% water, or even from 50wt% to 75wt% water.

Upon dissolution with deionized water at a temperature of 25°C and a dilution of 1g/l, the benefit delivery composition forms droplets, wherein the droplets have a volume average droplet size in the range of from 20$\mu$m to 1000$\mu$m, more preferably from 20$\mu$m to 500$\mu$m, or from 20$\mu$m to 150$\mu$m. Without wishing to be bound by theory, ensuring this droplet size distribution leads to good surface deposition, especially on fabric surfaces. Preferably, less than 50% by volume of the droplets have a droplet size greater than 150$\mu$m micrometers, more preferably less than 25%, or less than 10% or even less than 5% by volume of the droplets have a droplet size greater than 150$\mu$m. Without wishing to be bound by theory, by controlling the droplet size in this manner, the benefit delivery composition exhibits good surface deposition without the unwanted visual appearance of oily deposits on the surface. The method for measuring the droplet size of the benefit delivery composition is described in more detail below.

**Process for making a benefit delivery composition:** The process comprises the steps of:

(a) contacting a surfactant and a fatty amphiphile to form a lamellar phase composition;
(b) contacting the lamellar phase composition and a silicone to form the benefit delivery composition.

Steps (a) and (b) are described in more detail below.

The process can be a continuous process or can be a batch process. In a batch process, it may be preferred for steps (a) and (b) to be carried out in the same equipment. It may also be preferred for steps (a) and (b) to be carried out in separate equipment. For example, the lamellar phase composition is formed in a separate mixer to that used to dose the silicone.

**Step (a). Forming a lamellar phase composition:** During step (a), a surfactant is contacted to a fatty amphiphile to form a lamellar phase composition. During step (a), the fatty amphiphile is at a temperature above its melting point when it is contacted with the surfactant. Preferably, the surfactant is at a temperature above the melting point of the fatty amphiphile when it is contacted with the fatty amphiphile. If present, preferably the water is at a temperature above the melting point of the fatty amphiphile when it is contacted to the fatty amphiphile.

The surfactant and fatty amphiphile may be contacted at a temperature of at least 40°C, or even at least 70°C. Preferred heating means include hot water jacketing and/or hot oil jacketing. Other heating means include direct heat, electrical tracing, steam heating.

Suitable equipment for contacting the surfactant to the fatty amphiphile include mixers such as DPM range of

high torque mixers from Charles Ross & Son Company, Hauppauge, New York.

Preferably, step (a) is carried out at a pH in the range of from 4.0 to 7.0, more preferably from 5.0 to 6.0. When the fatty amphiphile is a fatty acid, preferably step (a) is carried out at a pH that corresponds to, or is similar to, the pKa of the fatty acid. When the fatty amphiphire is a fatty acid, preferably step (a) is carried out at a pH no greater than 0.5 pH units above the pKa of the fatty acid, and no less than 0.5 pH units below the pKa of the fatty acid.

**Step (b). Forming a benefit delivery composition:** During step (b), the lamellar phase composition is contacted to silicone to form the benefit delivery composition.

Step (b) is carried out under conditions of low shear, having a maximum tip speed of $2.5ms^{-1}$, preferably $2.0ms^{-1}$, or even $1.5ms^{-1}$, anda maximum shear rate of $500s^{-1}$, or from $400s^{-1}$ or even $300s^{-1}$. Without wishing to be bound by theory, carefully controlling the shear conditions in this manner result in a benefit delivery composition having a good surface deposition profile: high shear rates can lead to undesirably small droplet sizes of the resultant benefit delivery composition upon contact with water, which in turn lead to a poor deposition profile. Suitable equipment for carrying out step (b) include DPM range of high torque mixers from Charles Ross & Son Company, Hauppauge, New York.

**Lamellar phase composition:** The lamellar phase composition comprises surfactant and fatty amphiphile, preferably the lamellar phase composition comprises water. Preferably, the lamellar phase composition consists essentially only of surfactant, fatty amphile and water. Preferably, the molar ratio of surfactant to fatty amphiphile present in the lamellar phase composition is in the range of from 1:1 to 2.5:1, more preferably 1:1 to 1.5:1. Without wishing to be bound by theory, by controlling the molar ratio in this manner, the resultant droplet size of the benefit delivery composition upon contact with water can be controlled. Without wishing to be bound by theory, increasing the molar amount of fatty amphiphile relative to the molar amount of surfactant increases the resultant droplet size of the benefit deliver composition upon contact with water.

Preferably, the lamellar phase composition has a packing parameter in the range of from 0.5 to 1.0. The packing parameter and method for determining the packaging parameter is described in more detail below.

**Surfactant:** Suitable surfactants include anionic surfactants, non-ionic surfactants, zwitterionic surfactants and amphoteric surfactants.

Suitable anionic detersive surfactants include sulphate and sulphonate detersive surfactants. Suitable sulphonate detersive surfactants include alkyl benzene sulphonate, such as $C_{10-13}$ alkyl benzene sulphonate. Suitable alkyl benzene sulphonate (LAS) is obtainable, or even obtained, by sulphonating commercially available linear alkyl benzene (LAB); suitable LAB includes low 2-phenyl LAB, such as those supplied by Sasol under the tradename Isochem® or those supplied by Petresa under the tradename Petrelab®, other suitable LAB include high 2-phenyl LAB, such as those supplied by Sasol under the tradename Hyblene®. Another suitable anionic detersive surfactant is alkyl benzene sulphonate that is obtained by DETAL catalyzed process, although other synthesis routes, such as HF, may also be suitable.

Suitable sulphate detersive surfactants include alkyl sulphate, such as $C_{8-18}$ alkyl sulphate, or predominantly $C_{12}$ alkyl sulphate. The alkyl sulphate may be derived from natural sources, such as coco and/or tallow. Alternative, the alkyl sulphate may be derived from synthetic sources such as $C_{12-15}$ alkyl sulphate.

Another suitable sulphate detersive surfactant is alkyl alkoxylated sulphate, such as alkyl ethoxylated sulphate, or a $C_{8-18}$ alkyl alkoxylated sulphate, or a $C_{8-18}$ alkyl ethoxylated sulphate. The alkyl alkoxylated sulphate may have an average degree of alkoxylation of from 0.5 to 20, or from 0.5 to 10. The alkyl alkoxylated sulphate may be a $C_{8-18}$ alkyl ethoxylated sulphate, typically having an average degree of ethoxylation of from 0.5 to 10, or from 0.5 to 7, or from 0.5 to 5 or from 0.5 to 3.

The alkyl sulphate, alkyl alkoxylated sulphate and alkyl benzene sulphonates may be linear or branched, substituted or un-substituted.

The anionic detersive surfactant may be a mid-chain branched anionic detersive surfactant, such as a mid-chain branched alkyl sulphate and/or a mid-chain branched alkyl benzene sulphonate. The mid-chain branches are typically $C_{1-4}$ alkyl groups, such as methyl and/or ethyl groups.

Another suitable anionic detersive surfactant is alkyl ethoxy carboxylate.

The anionic detersive surfactants are typically present in their salt form, typically being complexed with a suitable cation. Suitable counter-ions include $Na^+$ and $K^+$.

Suitable non-ionic detersive surfactants are selected from the group consisting of: $C_8$-$C_{18}$ alkyl ethoxylates, such as, NEODOL® non-ionic surfactants from Shell; $C_6$-$C_{12}$ alkyl phenol alkoxylates wherein optionally the alkoxylate units are ethyleneoxy units, propyleneoxy units or a mixture thereof; $C_{12}$-$C_{18}$ alcohol and $C_6$-$C_{12}$ alkyl phenol condensates with ethylene oxide/propylene oxide block polymers such as Pluronic® from BASF; $C_{14}$-$C_{22}$ mid-chain branched alcohols; $C_{14}$-$C_{22}$ mid-chain branched alkyl alkoxylates, typically having an average degree of alkoxylation of from 1 to 30; alkylpolysaccharides, such as alkylpolyglycosides; polyhydroxy fatty acid amides; ether capped poly(oxyalkylated) alcohol surfactants; and mixtures thereof. Suitable nonionic detersive surfactants include secondary alcohol-based detersive surfactants. Other suitable non-ionic detersive surfactants include EO/PO block co-polymer surfactants, such as the Plurafac® series of surfactants available from BASF, and sugar-derived surfactants such as alkyl N-methyl glucose amide.

Preferred surfactants include alkyl benzene sulphonate, alkyl ethoxylated sulphate, and mixtures thereof. Pre-

ferred surfactants include $C_{10}$-$C_{13}$ alkyl benzene sulphonate, $C_{12}$-$C_{15}$ alkyl ethoxylated sulphate having an everage degree of ethoxylation in the range of from 1.0 to 5.0 and mixtures thereof. Preferably the surfactant is an anionic surfactant having a cationic counter-ion selected from sodium or calcium. Preferably, the surfactant has a HLB in the range of from 30 to 40.

**Fatty amphiphile:** Suitable fatty amphiphiles are selected from fatty acid, fatty alcohol and mixtures thereof. Preferred fatty amphiphiles are selected from $C_8$-$C_{16}$ fatty acid, $C_8$-$C_{16}$ fatty alcohol and mixtures thereof. A highly preferred fatty amphiphile is $C_{12}$ fatty acid.

Preferably, the fatty amphiphile has a melting point of at least 40°C, more preferably at least 50°C or even at least 60°C.

Preferably, the fatty amphiphile is a fatty acid having a pKa in the range of from 6 to 8. Preferably, the fatty amphiphile has a HLB in the range of from 10 to 20.

**Silicone:** Suitable silicones are selected from the group consisting of cyclic silicones, polydimethylsiloxanes, aminosilicones, cationic silicones, silicone polyethers, silicone resins, silicone urethanes, and mixtures thereof.

A preferred silicone is a polydialkylsilicone, alternatively a polydimethyl silicone (polydimethyl siloxane or "PDMS"), or a derivative thereof.

Preferably, the silicone has a viscosity at a temperature of 25°C and a shear rate of $1000s^{-1}$ in the range of from 10Pa s to 100Pa s. Without wishing to be bound by theory, increasing the viscosity of the silicone improves the deposition of the perfume onto the treated surface. However, without wishing to be bound by theory, if the viscosity is too high, it is difficult to process and form the benefit delivery composition. A preferred silicone is AK 60000 from Wacker, Munich, Germany

Other suitable silicones are selected from an aminofunctional silicone, amino-polyether silicone, alkyloxylated silicone, cationic silicone, ethoxylated silicone, propoxylated silicone, ethoxylated/propoxylated silicone, quaternary silicone, or combinations thereof.

Suitable silicones are selected from random or blocky organosilicone polymers having the following formula:

$$[R_1R_2R_3SiO_{1/2}]_{(j+2)}[(R_4Si(X\text{-}Z)O_{2/2}]_k[R_4R_4SiO_{2/2}]_m[R_4SiO_{3/2}]_j$$

wherein:

- j is an integer from 0 to about 98; in one aspect j is an integer from 0 to about 48; in one aspect, j is 0;
- k is an integer from 0 to about 200, in one aspect k is an integer from 0 to about 50; when k = 0, at least one of $R_1$, $R_2$ or $R_3$ is -X-Z;
- m is an integer from 4 to about 5,000; in one aspect m is an integer from about 10 to about 4,000; in another aspect m is an integer from about 50 to about 2,000;

$R_1$, $R_2$ and $R_3$ are each independently selected from the group consisting of H, OH, $C_1$-$C_{32}$ alkyl, $C_1$-$C_{32}$ substituted alkyl, $C_5$-$C_{32}$ or $C_6$-$C_{32}$ aryl, $C_5$-$C_{32}$ or $C_6$-$C_{32}$ substituted aryl, $C_6$-$C_{32}$ alkylaryl, $C_6$-$C_{32}$ substituted alkylaryl, $C_1$-$C_{32}$ alkoxy, $C_1$-$C_{32}$ substituted alkoxy and X-Z;

each $R_4$ is independently selected from the group consisting of H, OH, $C_1$-C32 alkyl, $C_1$-$C_{32}$ substituted alkyl, $C_5$-$C_{32}$ or $C_6$-$C_{32}$ aryl, $C_5$-$C_{32}$ or $C_6$-$C_{32}$ substituted aryl, $C_6$-$C_{32}$ alkylaryl, $C_6$-$C_{32}$ substituted alkylaryl, $C_1$-$C_{32}$ alkoxy and $C_1$-$C_{32}$ substituted alkoxy;

each X in said alkyl siloxane polymer comprises a substituted or unsubsitituted divalent alkylene radical comprising 2-12 carbon atoms, in one aspect each divalent alkylene radical is independently selected from the group consisting of $-(CH_2)_s-$ wherein s is an integer from about 2 to about 8, from about 2 to about 4; in one aspect, each X in said alkyl siloxane polymer comprises a substituted divalent alkylene radical selected from the group consisting of: $-CH_2-CH(OH)-CH_2-$; $-CH_2-CH_2-CH(OH)-$; and

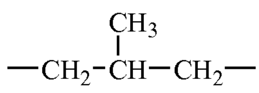

;

each Z is selected independently from the group consisting of

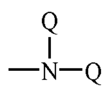

,

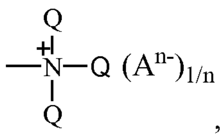,
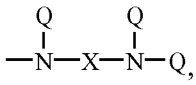,
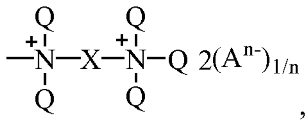,

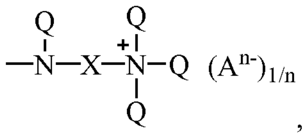,
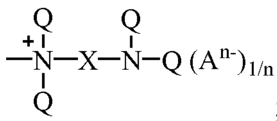;
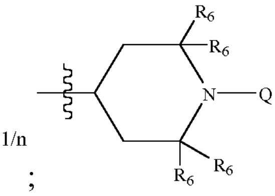

and

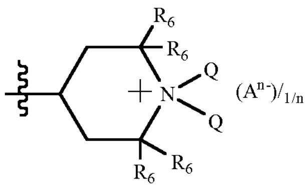

with the proviso that when Z is a quat, Q cannot be an amide, imine, or urea moiety and if Q is an amide, imine, or urea moiety, then any additional Q bonded to the same nitrogen as said amide, imine, or urea moiety must be H or a $C_1$-$C_6$ alkyl, in one aspect, said additional Q is H; for Z $A^{n-}$ is a suitable charge balancing anion. In one aspect $A^{n-}$ is selected from the group consisting of $Cl^-$, $Br^-$, $I^-$, methylsulfate, toluene sulfonate, carboxylate and phosphate ; and at least one Q in said organosilicone is independently selected from $-CH_2-CH(OH)-CH_2-R_5$;

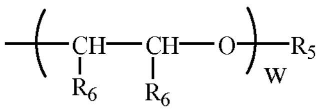;
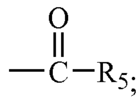;

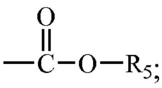;
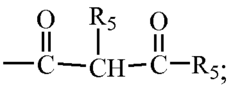;
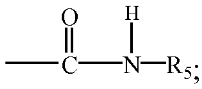;
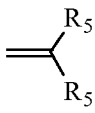;
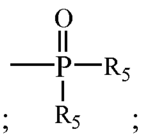;

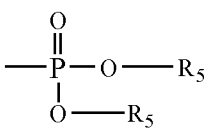;
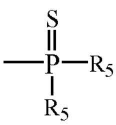;
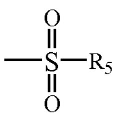;
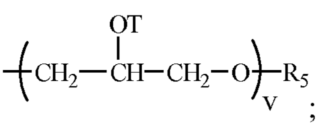;

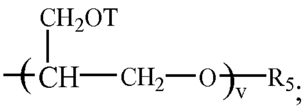;
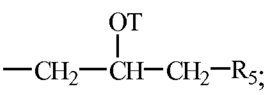; and
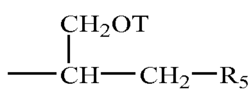

each additional Q in said organosilicone is independently selected from the group comprising of H, $C_1$-$C_{32}$ alkyl, $C_1$-$C_{32}$ substituted alkyl, $C_5$-$C_{32}$ or $C_6$-$C_{32}$ aryl, $C_5$-$C_{32}$ or $C_6$-$C_{32}$ substituted aryl, $C_6$-$C_{32}$ alkylaryl, $C_6$-$C_{32}$ substituted alkylaryl, $-CH_2-CH(OH)-CH_2-R_5$;

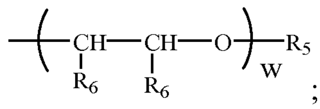 ; 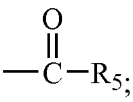 ;

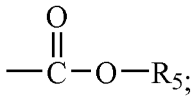 ; 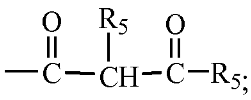 ; 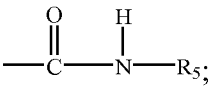 ; 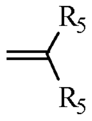 ; 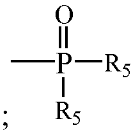

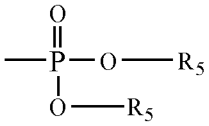 ; 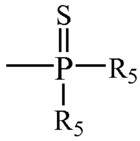 ; 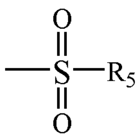 ; 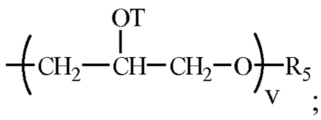 ;

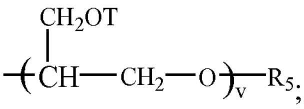 ; 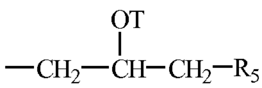 and 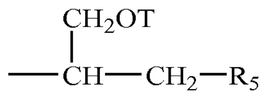

wherein each $R_5$ is independently selected from the group consisting of H, $C_1$-$C_{32}$ alkyl, $C_1$-$C_{32}$ substituted alkyl, $C_5$-$C_{32}$ or $C_6$-$C_{32}$ aryl, $C_5$-$C_{32}$ or $C_6$-$C_{32}$ substituted aryl, $C_6$-$C_{32}$ alkylaryl, $C_6$-$C_{32}$ substituted alkylaryl, $-(CHR_6-CHR_6-O-)_w-L$ and a siloxyl residue; each $R_6$ is independently selected from H, $C_1$-$C_{18}$ alkyl each L is independently selected from $-C(O)-R_7$ or
$R_7$;
w is an integer from 0 to about 500, in one aspect w is an integer from about 1 to about 200; in one aspect w is an integer from about 1 to about 50; each $R_7$ is selected independently from the group consisting of H; $C_1$-$C_{32}$ alkyl; $C_1$-$C_{32}$ substituted alkyl, $C_5$-$C_{32}$ or $C_6$-$C_{32}$ aryl, $C_5$-$C_{32}$ or $C_6$-$C_{32}$ substituted aryl, $C_6$-$C_{32}$ alkylaryl; $C_6$-$C_{32}$ substituted alkylaryl and a siloxyl residue; each T is independently selected from H, and

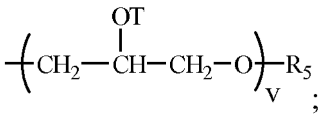 ;

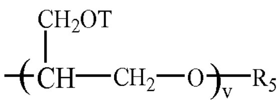 ; 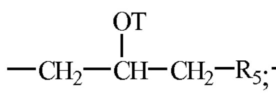 ; 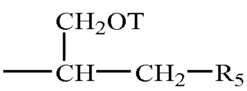

and wherein each v in said organosilicone is an integer from 1 to about 10, in one aspect, v is an integer from 1 to about 5 and the sum of all v indices in each Q in the said organosilicone is an integer from 1 to about 30 or from 1 to about 20 or even from 1 to about 10.

In another embodiment, the silicone may be chosen from a random or blocky organosilicone polymer having the following formula:

$$[R_1R_2R_3SiO_{1/2}]_{(j+2)}[(R_4Si(X\text{-}Z)O_{2/2}]_k[R_4R_4SiO_{2/2}]_m[R_4SiO_{3/2}]_j$$

wherein

j is an integer from 0 to about 98; in one aspect j is an integer from 0 to about 48; in one aspect, j is 0;
k is an integer from 0 to about 200; when k = 0, at least one of $R_1$, $R_2$ or $R_3$ = -X-Z, in one aspect, k is an integer from 0 to about 50
m is an integer from 4 to about 5,000; in one aspect m is an integer from about 10 to about 4,000; in another aspect m is an integer from about 50 to about 2,000;

$R_1$, $R_2$ and $R_3$ are each independently selected from the group consisting of H, OH, $C_1$-$C_{32}$ alkyl, $C_1$-$C_{32}$ substituted alkyl, $C_5$-$C_{32}$ or $C_6$-$C_{32}$ aryl, $C_5$-$C_{32}$ or $C_6$-$C_{32}$ substituted aryl, $C_6$-$C_{32}$ alkylaryl, $C_6$-$C_{32}$ substituted alkylaryl, $C_1$-$C_{32}$ alkoxy, $C_1$-$C_{32}$ substituted alkoxy and X-Z;

each $R_4$ is independently selected from the group consisting of H, OH, $C_1$-$C_{32}$ alkyl, $C_1$-$C_{32}$ substituted alkyl, $C_5$-$C_{32}$ or $C_6$-$C_{32}$ aryl, $C_5$-$C_{32}$ or $C_6$-$C_{32}$ substituted aryl, $C_6$-$C_{32}$ alkylaryl, $C_6$-$C_{32}$ substituted alkylaryl, $C_1$-$C_{32}$ alkoxy and $C_1$-$C_{32}$ substituted alkoxy;

each X comprises of a substituted or unsubstituted divalent alkylene radical comprising 2-12 carbon atoms; in one aspect each X is independently selected from the group consisting of - $(CH_2)_s$-O-; $-CH_2-CH(OH)-CH_2-O-$;

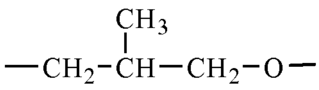 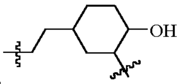

and

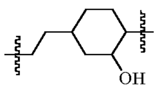

wherein each s independently is an integer from about 2 to about 8, in one aspect s is an integer from about 2 to about 4; At least one Z in the said organosiloxane is selected from the group consisting of $R_5$;

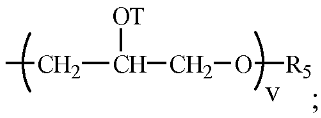 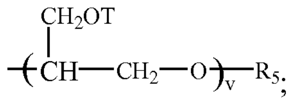 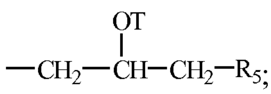

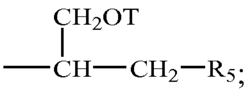 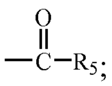 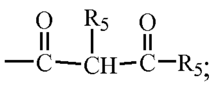 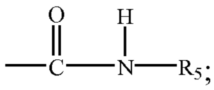 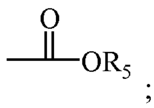

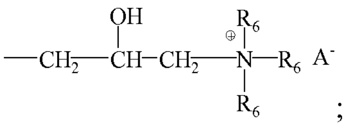 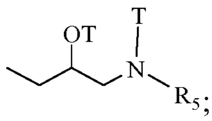 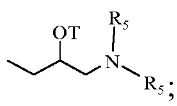

$-C(R_5)_2O-R_5$;

$-C(R_5)_2S-R_5$ and 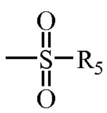

provided that when X is

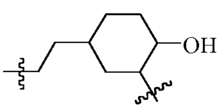 or 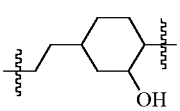

then Z = $-OR_5$ or

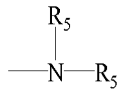

wherein $A^-$ is a suitable charge balancing anion. In one aspect $A^-$ is selected from the group consisting of $Cl^-$, $Br^-$, $I^-$, methylsulfate, toluene sulfonate, carboxylate and phosphate and each additional Z in said organosilicone is independently selected from the group comprising of H, $C_1$-$C_{32}$ alkyl, $C_1$-$C_{32}$ substituted alkyl, $C_5$-$C_{32}$ or $C_6$-$C_{32}$ aryl, $C_5$-$C_{32}$ or $C_6$-$C_{32}$ substituted aryl, $C_6$-$C_{32}$ alkylaryl, $C_6$-$C_{32}$ substituted alkylaryl, $R_5$,

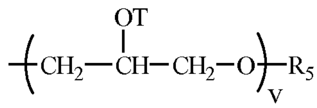

; 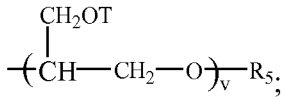 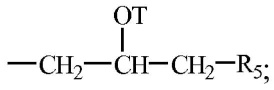 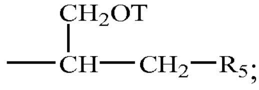 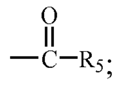

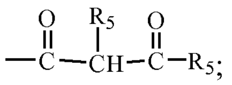 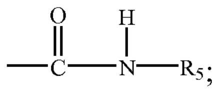 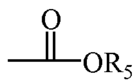 ; 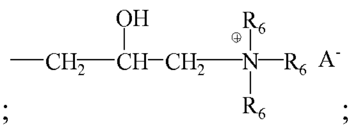 ;

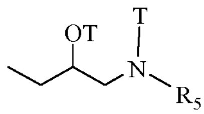 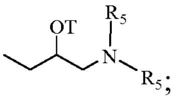 $-C(R_5)_2O-R_5; -C(R_5)_2S-R_5$ and 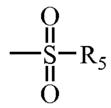

provided that when X is

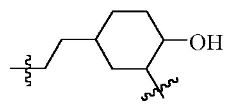 or 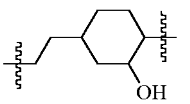

then Z = $-OR_5$ or

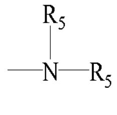

each $R_5$ is independently selected from the group consisting of H; $C_1$-$C_{32}$ alkyl; $C_1$-$C_{32}$ substituted alkyl, $C_5$-$C_{32}$ or $C_6$-$C_{32}$ aryl, $C_5$-$C_{32}$ or $C_6$-$C_{32}$ substituted aryl or $C_6$-$C_{32}$ alkylaryl, or $C_6$-$C_{32}$ substituted alkylaryl, $-(CHR_6-CHR_6-O-)_w-CHR_6-CHR_6-L$ and siloxyl residue wherein each L is independently selected from $-O-C(O)-R_7$ or $-O-R_7$;

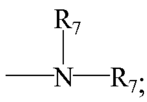 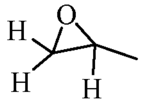 and 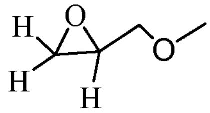

w is an integer from 0 to about 500, in one aspect w is an integer from 0 to about 200, one aspect w is an integer from 0 to about 50;

each $R_6$ is independently selected from H or $C_1$-$C_{18}$ alkyl;
each $R_7$ is independently selected from the group consisting of H; $C_1$-$C_{32}$ alkyl; $C_1$-$C_{32}$ substituted alkyl, $C_5$-$C_{32}$ or $C_6$-$C_{32}$ aryl, $C_5$-$C_{32}$ or $C_6$-$C_{32}$ substituted aryl, $C_6$-$C_{32}$ alkylaryl, and $C_6$-$C_{32}$ substituted aryl, and a siloxyl residue;
each T is independently selected from H;

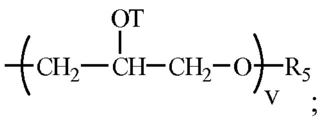 ; 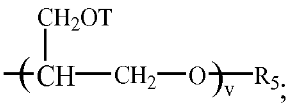 ;

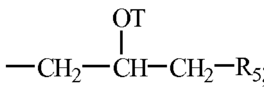 ; 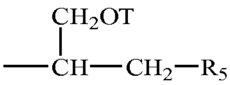

wherein each v in said organosilicone is an integer from 1 to about 10, in one aspect, v is an integer from 1 to about 5 and the sum of all v indices in each Z in the said organosilicone is an integer from 1 to about 30 or from 1 to about 20 or even from 1 to about 10.

A suitable silicone is a blocky cationic organopolysiloxane having the formula:

$$M_wD_xTyQ_z$$

wherein:

M = $[SiR_1R_2R_3O_{1/2}]$, $[SiR_1R_2G_1O_{1/2}]$, $[SiR_1G_1G_2O_{1/2}]$, $[SiG_1G_2G_3O_{1/2}]$, or combinations thereof;
D = $[SiR_1R_2O_{2/2}]$, $[SiR_1G_1O_{2/2}]$, $[SiG_1G_2O_{2/2}]$ or combinations thereof;
T = $[SiR_1O_{3/2}]$, $[SiG_1O_{3/2}]$ or combinations thereof;
Q = $[SiO_{4/2}]$;
w = is an integer from 1 to (2+y+2z);
x = is an integer from 5 to 15,000;
y = is an integer from 0 to 98;
z = is an integer from 0 to 98;
$R_1$, $R_2$ and $R_3$ are each independently selected from the group consisting of H, OH, $C_1$-$C_{32}$ alkyl, $C_1$-$C_{32}$ substituted alkyl, $C_5$-$C_{32}$ or $C_6$-$C_{32}$ aryl, $C_5$-$C_{32}$ or $C_6$-$C_{32}$ substituted aryl, $C_6$-$C_{32}$ alkylaryl, $C_6$-$C_{32}$ substituted alkylaryl, $C_1$-$C_{32}$ alkoxy, $C_1$-$C_{32}$ substituted alkoxy, $C_1$-$C_{32}$ alkylamino, and $C_1$-$C_{32}$ substituted alkylamino;
at least one of M, D, or T incorporates at least one moiety $G_1$, $G_2$ or $G_3$; and $G_1$, $G_2$, and $G_3$ are each independently selected from the formula:

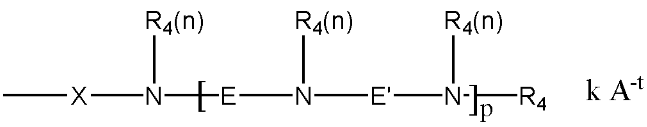

wherein:

X comprises a divalent radical selected from the group consisting of $C_1$-$C_{32}$ alkylene, $C_1$-$C_{32}$ substituted alkylene, $C_5$-$C_{32}$ or $C_6$-$C_{32}$ arylene, $C_5$-$C_{32}$ or $C_6$-$C_{32}$ substituted arylene, $C_6$-$C_{32}$ arylalkylene, $C_6$-$C_{32}$ substituted arylalkylene, $C_1$-$C_{32}$ alkoxy, $C_1$-$C_{32}$ substituted alkoxy, $C_1$-$C_{32}$ alkyleneamino, $C_1$-$C_{32}$ substituted alkyleneamino, ring-opened epoxide, and ring-opened glycidyl, with the proviso that if X does not comprise a repeating alkylene oxide moiety then X can further comprise a heteroatom selected from the group consisting of P, N and O;

each $R_4$ comprises identical or different monovalent radicals selected from the group consisting of H, $C_1$-$C_{32}$ alkyl, $C_1$-$C_{32}$ substituted alkyl, $C_5$-$C_{32}$ or $C_6$-$C_{32}$ aryl, $C_5$-$C_{32}$ or $C_6$-$C_{32}$ substituted aryl, $C_6$-$C_{32}$ alkylaryl, and $C_6$-$C_{32}$ substituted alkylaryl;

E comprises a divalent radical selected from the group consisting of $C_1$-$C_{32}$ alkylene, $C_1$-$C_{32}$ substituted alkylene, $C_5$-$C_{32}$ or $C_6$-$C_{32}$ arylene, $C_5$-$C_{32}$ or $C_6$-$C_{32}$ substituted arylene, $C_6$-$C_{32}$ arylalkylene, $C_6$-$C_{32}$ substituted arylalkylene, $C_1$-$C_{32}$ alkoxy, $C_1$-$C_{32}$ substituted alkoxy, $C_1$-$C_{32}$ alkyleneamino, $C_1$-$C_{32}$ substituted alkyleneamino,

ring-opened epoxide and ring-opened glycidyl, with the proviso that if E does not comprise a repeating alkylene oxide moiety then E can further comprise a heteroatom selected from the group consisting of P, N, and O;

E' comprises a divalent radical selected from the group consisting of $C_1$-$C_{32}$ alkylene, $C_1$-$C_{32}$ substituted alkylene, $C_5$-$C_{32}$ or $C_6$-$C_{32}$ arylene, $C_5$-$C_{32}$ or $C_6$-$C_{32}$ substituted arylene, $C_6$-$C_{32}$ arylalkylene, $C_6$-$C_{32}$ substituted arylalkylene, $C_1$-$C_{32}$ alkoxy, $C_1$-$C_{32}$ substituted alkoxy, $C_1$-$C_{32}$ alkyleneamino, $C_1$-$C_{32}$ substituted alkyleneamino, ring-opened epoxide and ring-opened glycidyl, with the proviso that if E' does not comprise a repeating alkylene oxide moiety then E' can further comprise a heteroatom selected from the group consisting of P, N, and O;

p is an integer independently selected from 1 to 50;

n is an integer independently selected from 1 or 2;

when at least one of $G_1$, $G_2$, or $G_3$ is positively charged, $A^{-t}$ is a suitable charge balancing anion or anions such that the total charge, k, of the charge-balancing anion or anions is equal to and opposite from the net charge on the moiety $G_1$, $G_2$ or $G_3$; wherein t is an integer independently selected from 1, 2, or 3; and $k \leq (p*2/t) + 1$; such that the total number of cationic charges balances the total number of anionic charges in the organopolysiloxane molecule;

and wherein at least one E does not comprise an ethylene moiety.

**Other ingredients:** Suitable ingredients are selected from petrolatum and/or sensate. Suitable sensates are compounds that provide a cooling, warming, tingling or refreshing sensation, either through the endothermic or exothermic processes of physical lowering or raising of temperature; or through the physiological cooling process associated with, e.g., cold menthol receptor (TRPM8), or any other receptors generally located at or near nerve endings. Suitable sensates include menthol and derivatives thereof. Suitable menthol derivatives include menthyl lactate (available under the trade name Frescolat ML from Symrise GmbH & Co., Holzminden, Germany), menthol with a carboxamide derivative, menthol with a cyclohexanecarboxamide derivative, dimethyl menthyl succinimide, menthone glycerin acetal (available under the trade name Frescolat MGA from Symrise GmbH & Co., Holzminden, Germany), menthoxypropanediol (commercially available under the trade name Coolact 10 and Coolact P (-)-isopulegol from Takasago Int'l Corp., Tokyo, Japan); neoisomenthol, neomenthol, isomenthol, PMD 38 p-menthane-3,8,-diol, (2R)-3-(1-menthoxy)propane-1,2-diol, (2RS)-3-(1-menthoxy)propane-1,2-diol; N-ethyl-p-menthane-3-carboxamide (WS-3), ethyleneglycol p-menthane-3-carboxylate (WS-4), ethyl 3-(p-menthane-3-carboxamido)acetate (WS-5), N-(4-methoxyphenyl)-p-menthane-3-carboxamide (WS-12), N-t-butyl-p-menthane-carboxamide (WS-14),2-isopropyl-N-2,3-trimethylbutyramide (WS-23), 1-glyceryl p-menthane-3-carboxylate (WS-30) (all commercially available from Millennium Chemicals, Hunt Valley, MD, USA). Other suitable sensates include phenol derivatives, such as thymol and eugenol, Icilin (Phoenix Pharmaceuticals, Belmont, CA, USA), 2(5H)-MPF (Nestec, Vevey, Switzerland), 4-methyl-3-(1-pyrrolidinyl)2[5H]-furanone, MPD vanillyl acetal (Takasago Int'l Corp., Tokyo, Japan) Hotact VBE (Lipo Chemicals, Inc., Paterson, NJ, USA) and capsaicin (derivative of cayenne pepper).

**Application of the benefit delivery composition:** The benefit delivery composition can be incorporated into a variety of consumer goods products, such as laundry detergent compositions, dish-washing detergent compositions, hard surface cleaning compositions, fabric enhancer compositions, shampoo, hair conditioners, skin creams, skin lotions, razer strips and cartridge compositions, shaving creams, foams and gels, body wash compositions, and dentifrice compositions.

The benefit delivery composition is contacted with at least three different consumer goods products ingredients to form the consumer goods product. Suitable consumer goods product ingredients are those typically found in the consumer goods product. For example, enzymes, bleach, polymers for detergent consumer goods products.

**Packing Parameter:** The surfactant Packing Parameter (N), is calculated from various molecular descriptors of the surfactant molecule's chemical structure, as described in more detail below. The surfactant Packing Parameter (N) is defined as:

$$N = v / l\, a_0$$

wherein,

v is the volume of the hydrocarbon core in cubic nanometers,

l is the length of the hydrocarbon chains, and
ao is the area of the surfactant head-group at the interface of the hydrophobic core.

The volume of the hydrocarbon core of a saturated chain (v), in cubic nanometers, is determined according to the following equation:

$$v = 0.027\ (n_c + n_{Me})$$

wherein,

$n_c$ is the total number of carbon atoms per chain, and
$n_{Me}$ is the number of methyl groups which are twice the size of a $CH_2$ group.

The maximum length of a fully extended hydrocarbon chain (1) (in nanometers) is calculated according to the following equation:

$$l = 0.15 + 0.127\ \ n_c$$

wherein,
$n_c$ is the total number of carbon atoms per chain.

The 0.15 nm in this equation comes from van der Waals radius of the terminal methyl group (0.21 nm) minus half the bond length of the first atom not contained in the hydrocarbon core (0.06 nm). The 0.127 nm is the carbon-carbon bond length (0.154 nm) projected onto the direction of the chain in the all-trans configuration.

The area of the surfactant head-group at the interface of the hydrophobic core (ao), is deteremined according to the calculations described in the following published article: " Theory of Self-Assembly of Hydrocarbon Amphiphiles into Micelles and Bilayers" 1976, J. Chem. Soc., Faraday Trans. 2, 72, 1525-1568, Jacob N. Israelachvili, D. John Mitchell and Barry W. Ninham.

**ClogP:** The logP values of many perfume materials have been reported; for example, the Pomona92 database, available from Daylight Chemical Information Systems, Inc. (Daylight CIS, Irvine, California), contains many, along with citations to the original literature. However, the logP values are most conveniently calculated by the "CLOGP" program, also available from Daylight CIS. The "calculated logP" (ClogP) is determined by the fragment approach of Hansch and Leo ( cf., A. Leo, in Comprehensive Medicinal Chemistry, Vol. 4, C. Hansch, P. G. Sammens, J. B. Taylor and C. A. Ramsden, Eds., p. 295, Pergamon Press, 1990). The fragment approach is based on the chemical structure of each perfume ingredient, and takes into account the numbers and types of atoms, the atom connectivity, and chemical bonding.

**Boiling Point:** The boiling point of perfume material is measured according to standard test method ASTM D2887-04a, "Standard Test Method for Boiling Range Distribution of Petroleum Fractions by Gas Chromatography," (ASTM International, West Conshohocken, Pennsylvania, USA".

**Melting Point:** The Melting Point value is determined using the widely used standard Differential Scanning Calorimetry methodology described in the following published article: "Comprehensive Evaluation of the Melting Points of Fatty Acids and Esters Determined by Differential Scanning Calorimetry". J. Am. Oil Chem. Soc. (2009). 86:843-856A.

**pKa:** The pKa value is the negative log (base 10) of the acid dissociation constant. The acid dissociation constant, $K_a$, is the equilibrium constant for the acid-base dissociation reaction.
The equilibrium of acid dissociation can be written symbolically as:

$$HA \rightleftharpoons A^- + H^+$$

where HA is a generic acid that dissociates by splitting into $A^-$, known as the conjugate base of the acid, and the hydrogen ion or proton, $H^+$. The dissociation constant is usually written as a quotient of the equilibrium concentrations (in mol/L), denoted by [HA], $[A^-]$ and $[H^+]$:

$$K_a = \frac{[A^-][H^+]}{[HA]}$$

The logarithmic constant, $pK_a$, which is equal to $-\log_{10} K_a$, is sometimes also referred to as an acid dissociation constant:

$$pK_a = -\log_{10} K_a$$

**HLB:** Hydrophilic-Lipophilic Balance (HLB) values are calculated according to the widely used standard methodology contained in the following published article: "The HLB System", 1987, ICI Americas Inc., Wilmington, Delaware, USA.

**Method for measuring droplet size:** A Malvern Mastersizer 2000 (Malvern Instruments GmbH, Rigipsstr. 19 Herrenberg 71083 Germany), is used to measure the droplet size of the lamellar gel sample dispersed in filtered deionized (DI) water. The Mastersizer 2000 uses the technique of laser diffraction to measure the size of particles. It does this by measuring the intensity of light scattered as a laser beam passes through a dispersed particulate sample. The sample dispersion is prepared by dissolving 1 g of the lamellar gel sample in 0.8 L filtered deionized water at 40 °C, with a mixing speed of 1500 rpm. If this concentration is not sufficient to achieve 20% obscuration in the Mastersizer instrument using the following settings, then additional sample material is added in order to achieve 20% obscuration. The change in droplet size is monitored over a period of 30 minutes. This data is then analyzed to calculate the size of the particles that created the scattering pattern, using the following two refractive indices: 1.333 (dispersant = water), and 1.469 (hydrophobic active materials), and the adsorption parameter of the system is set to 1. The average droplet size is expressed as the mean volume average diameter.

**Method for measuring viscosity:** The viscosity is measured by the following method, which generally represents the zero-shear viscosity (or zero-rate viscosity). Viscosity measurements are made with an AR2000 Controlled-Stress Rheometer (TA Instruments, New Castle, Delaware, U.S.A.), and accompanying software version 5.7.0. The instrument is outfitted with a 40 mm stainless steel parallel plate (TA Instruments catalog no. 511400.901) and Peltier plate (TA Instruments catalog no. 533230.901). The calibration is done in accordance with manufacturer recommendations. A refrigerated, circulating water bath set to 25 °C is attached to the Peltier plate.

Measurements are made on the instrument with the following procedures: Conditioning Step (pre-condition the sample) under "Settings" label, initial temperature: 25 °C, pre-shear at 5.0 $s^{-1}$ for 1 minute, equilibrate for 2 minutes; Flow-Step (measure viscosity) under "Test" Label, Test Type: "Steady State Flow", Ramp: "shear rate 1/s" from 0.001 $s^{-1}$ and 1000 $s^{-1}$, Mode: "Log", Points per Decade: 15, Temperate: 25 °C, Percentage Tolerance: 5, Consecutive with Tolerance: 3, Maximum Point Time: 45 sec, Gap set to 1000 micrometers, Stress-Sweep Step is not checked; Post-Experiment Step under "Settings" label; Set temperature: 25 °C.

More than 1.25 ml of the test sample of the component to be measured is dispensed through a pipette on to the center of the Peltier plate. The 40 mm plate is slowly lowered to 1100 micrometers, and the excess sample is trimmed away from the edge of the plate with a rubber policeman trimming tool or equivalent. Lower the plate to 1000 micrometers (gap setting) prior to collecting the data.

Discard any data points collected with an applied rotor torque of less than 1 micro-N·m (e.g. discard data less than ten-fold the minimum torque specification). Create a plot of viscosity versus shear rate on a log-log scale. These plotted data points are analyzed in one of three ways to determine the viscosity value:

first, if the plot indicates that the sample is Newtonian, in that all viscosity values fall on a plateau within +/- 20% of the viscosity value measured closest to 1 micro-N·m, then the viscosity is determined by fitting the 'Newtonian' fit model in the software to all the remaining data;

second, if the plot reveals a plateau in which the viscosity does not change by +/- 20% at low shear rates and a sharp, nearly-linear decrease in viscosity in excess of the +/- 20% at higher shear rates, then the viscosity is determined by applying the "Best Fit Using Viscosity vs. Rate" option from the "Analysis Toolbar";

third, if the plot indicates that the sample is only shear-thinning, in that there is only a sharp, nearly-linear decrease in viscosity, then the material is characterized by a viscosity which is taken as the largest viscosity in the plotted data, generally a viscosity measured close to 1 micro-N·m of applied torque.

Report the average value of the replicates as the viscosity of the component, in units of Pa·s.

EXAMPLES

**EXAMPLE 1:** The following samples are prepared by the processes described below. Sample 2 is in accordance with the present invention. Sample 3 is a comparison example where the fatty amphiphile (dodecanoic acid) is not melted when it is contacted to the LAS.

| Ingredients | | Sample 2 In accordance with the present invention | Sample 3 Comparison example (fatty amphiphile not melted) |
|---|---|---|---|
| **Neutralised LAS paste (45% active)** | | 36.690wt% | 36.690wt% |
| **Dodecanoic acid** | | 9.490wt% | 9.490wt% |
| **Water** | | 18.642wt% | 18.642wt% |
| **$Na_2CO_3$** | | 0.098wt% | 0.098wt% |
| **NaHCO3** | | 0.080wt% | 0.080wt% |
| **PDMS** | | 0wt% | 0wt% |
| **Perfume** | | 0wt% | 0wt% |
| **Silicone/Perfume Pre Mix** | | 35.000wt% | 35.000wt% |
| **Total** | | 100.000wt% | 100.000wt% |

**Process of making the samples:**

**LAS paste neutralization:** 72.38g of LAS paste (45% active) is heated to 60°C and continuously stirred at 1000 rpm in a heat resistant beaker and HCl (10 M) is added drop wise until a pH of 7.0 is obtained. The LAS paste is then stored in an oven at 50°C tightly sealed to avoid water evaporation.

**Process of making sample 2 (in accordance with the present invention):** 18.98g dodecanoic acid is placed in a plastic container in an oven at 50°C (above its melting point of 43.2°C). A stirrer blade is warmed in the oven at 50 °C for at least one hour and then the blade is placed and locked in an overhead stirrer. 72.38g LAS Paste (prepared as described above) is shaken vigourously and dosed into the overhead stirrer. 18.98g molten dodecanoic acid (prepared as described above) is added to the overhead stirrer and the mixture is stirred at 50°C, 350rpm for 5 minutes to form a gel. 0.196g sodium carbonate and 0.160g sodium bicarbonate are added to 37.284g deionized water and mixed to form a buffer. 37.64g buffer is heated to 50°C and added to the gel (prepared as described above) and stirred for 5 minutes at 350rpm. The gel is then cooled to room temperature. 60g silicone (PDMS) and 10g perfume are mixed in a high speed mixer (Siemens Speed Mixer DAC150FVZK) at 2700 rpm for 3 minutes to form a premix.The premix is then added to the gel (prepared as described above) and the gel is stirred at room temperature for 15 minutes at 350 RPM.

**Process of making Sample 3 (comparison example, fatty amphiphile not melted):** 18.98g dodecanoic acid is placed in a plastic container at room temperature (20°C). A stirrer blade at room temperature is placed and locked in an overhead stirrer. 72.38g LAS Paste (prepared as described above) is cooled to room temperature, shaken vigourously, and dosed into the overhead stirrer. 18.98g dodecanoic acid (prepared as described above) is added to the overhead stirrer and the mixture is stirred at room temperature, 350rpm for 5 minutes to form a gel. 0.196g sodium carbonate and 0.160g sodium bicarbonate are added to 37.284g deionized water and mixed to form a buffer. 37.64g buffer is then added to the gel (prepared as described above) at room temperature and stirred for 5 minutes at 350rpm. 60g silicone (PDMS) and 10g perfume are mixed in a high speed mixer (Siemens Speed Mixer DAC150FVZK) at 2700 rpm for 3 minutes to form a premix.The premix is then added to the gel (prepared as described above) and the gel is stirred at room temperature for 15 minutes at 350 RPM.

**Test protocol:** Each of the above described samples 2 and 3 were tested for softening, freshness and spotting performance on fabric using the following test protocol.

**Freshness, softening, and spotting performance method:**

The samples were added into a mini washing system along with a laundry detergent (Ariel UK unscented laundry powder). The mini washing system is a 8L water volume mini replica of a top loading automatic washing machine. The hardness of the water used was 8 gpg (54.88mg calcium/L).

The following fabrics are added into mini-washer pots: 3 x Christy Softness Swatches 20cm x 20cm; 2 x 1/8th Tonrose Towel 6.25cm x 12.5cm; 1 x Asda Poly-cotton Sheet 25cm x25 cm. These fabrics are supplied by Asda Stores Ltd., Leeds, UK or Optima Cotton Wear 8050 East Crystal Drive, Anaheim, CA 92807. The loaded mini-washer pots are agitated for 30 seconds and 60g laundry detergent (Ariel unscented laundry powder, UK) and 2.3g sample are then added to the miniwasher pot. A reference leg of 60g unscented laundry detergent (Ariel unscented laundry powder UK)

and 0.12g perfume oil was also placed in one of the mini-washer pots. The mini-washer then performed a 12min wash cycle, 2min spin cycle, 2min rinse cycle and a further 2min spin cycle. The treated fabric are dried at 21°C, 55% relative humidity for 15 hours. The fabrics are then graded to assess the fabric's softeness, freshness and spotting characteristics.

**Softeness paneling:** Panel grading is used to assess the softness characteristics. The panelists are trained and calibrated and panel the fabrics versus the reference fabric using the following panel score units (PSU) where -4 is described as significantly very poor versus reference, -3 is poor versus reference, -2 is slightly poor versus reference, -1 is unsure about negative difference versus reference, 0 is no difference versus reference, +1 is unsure about positive difference versus reference, +2 is slightly better versus reference, +3 is suprerior verus reference and +4 is significantly superior versus reference. Four replica fabrics are prepared for each sample, and each fabric is paneled once by three different panelists and the average panel score is calculated.

**Spotting performance:** Spotting performance is evaluated by counting the number of oily spots visibly observed per $cm^2$ on the polycotton fabrics. Two replica fabrics are prepared for each sample, and each fabric is evaluated by one panelist.

**Freshness performance:** Panel grading is used to assess the freshness characteristics. The panelists are trained and calibrated and panel the fabrics versus the reference fabric using the following primavera scale where +2.5 indicates a meaningful but not consumer noticeable positive difference versus reference, +5.0 indicates a meaningful and consumer noticeable positive difference versus reference, and +7.5 indicates a meaningful and highly consumer noticeable positive difference versus reference. A difference of 2.5 is considered to be a technical difference on the primavera scale. Four replica fabrics are prepared for each sample, and each fabric is paneled by two different panelists.

| | **Softeness performance (PSU)** | **Spotting performance (# spots / $cm^2$)** | **Freshness performance (primavera delta)** |
|---|---|---|---|
| | | | |
| **Sample 2** | +3 | 0.2 | +7.5 |
| **Sample 3** | +0.5 | 1.25 | +5.0 |

Sample 2 (in accordance with the present invention) has superior softeness performance and freshness performance, and showed the least amount of spotting compared to the comparison example samples 3.

**Example 2: Applications of the benefit delivery composition.**

**Solid free-flowing particulate laundry detergent composition examples:**

| **Ingredient** | **Amount (in wt%)** |
|---|---|
| **Benefit delivery composition of the present invention** (such as sample 2) | from 3wt% to 48wt% |
| **Anionic detersive surfactant** (such as alkyl benzene sulphonate, alkyl ethoxylated sulphate and mixtures thereof) | from 8wt% to 15wt% |
| **Non-ionic detersive surfactant** (such as alkyl ethoxylated alcohol) | from 0.5wt% to 4wt% |
| **Cationic detersive surfactant** (such as quaternary ammonium compounds) | from 0 to 4wt% |
| **Other detersive surfactant** (such as zwiterionic detersive surfactants, amphoteric surfactants and mixtures thereof) | from 0wt% to 4wt% |
| **Carboxylate polymer** (such as co-polymers of maleic acid and acrylic acid | from 1wt% to 4wt% |
| **Polyethylene glycol polymer** (such as a polyethylene glycol polymer comprising polyvinyl acetate side chains) | from 0.5wt% to 4wt% |
| **Polyester soil release polymer** (such as Repel-o-tex and/or Texcare polymers) | from 0.1 to 2wt% |
| **Cellulosic polymer** (such as carboxymethyl cellulose, methyl cellulose and combinations thereof) | from 0.5wt% to 2wt% |
| **Other polymer** (such as care polymers) | from 0wt% to 4wt% |

(continued)

| Ingredient | Amount (in wt%) |
|---|---|
| **Zeolite builder and phosphate builder** (such as zeolite 4A and/or sodium tripolyphosphate) | from 0wt% to 4wt% |
| **Other co-builder** (such as sodium citrate and/or citric acid) | from 0wt% to 3wt% |
| **Carbonate salt** (such as sodium carbonate and/or sodium bicarbonate) | from 0wt% to 15wt% |
| **Silicate salt** (such as sodium silicate) | from 0wt% to 10wt% |
| **Filler** (such as sodium sulphate and/or bio-fillers) | from 10wt% to 50wt% |
| **Source of hydrogen peroxide** (such as sodium percarbonate) | from 0wt% to 20wt% |
| **Bleach activator** (such as tetraacetylethylene diamine (TAED) and/or nonanoyloxybenzenesulphonate (NOBS)) | from 0wt% to 8wt% |
| **Bleach catalyst** (such as oxaziridinium-based bleach catalyst and/or transition metal bleach catalyst) | from 0wt% to 0.1wt% |
| **Other bleach** (such as reducing bleach and/or pre-formed peracid) | from 0wt% to 10wt% |
| **Photobleach** (such as zinc and/or aluminium sulphonated phthalocyanine) | from 0wt% to 0.1wt% |
| **Chelant** (such as ethylenediamine-N'N'-disuccinic acid (EDDS) and/or hydroxyethane diphosphonic acid (HEDP)) | from 0.2wt% to 1wt% |
| **Hueing agent** (such as direct violet 9, 66, 99, acid red 50, solvent violet 13 and any combination thereof) | from 0wt% to 1wt% |
| **Brightener** (C.I. fluorescent brightener 260 or C.I. fluorescent brightener 351) | from 0.1wt% to 0.4wt% |
| **Protease** (such as Savinase, Savinase Ultra, Purafect, FN3, FN4 and any combination thereof) | from 0.1wt% to 0.4wt% |
| **Amylase** (such as Termamyl, Termamyl ultra, Natalase, Optisize, Stainzyme, Stainzyme Plus and any combination thereof) | from 0.05wt% to 0.2wt% |
| **Cellulase** (such as Carezyme and/or Celluclean) | from 0.05wt% to 0.2wt% |
| **Lipase** (such as Lipex, Lipolex, Lipoclean and any combination thereof) | from 0.1 to 1wt% |
| **Other enzyme** (such as xyloglucanase, cutinase, pectate lyase, mannanase, bleaching enzyme) | from 0wt% to 2wt% |
| **Fabric softener** (such as montmorillonite clay and/or polydimethylsiloxane (PDMS)) | |
| **Flocculant** (such as polyethylene oxide) | from 0wt% to 1wt% |
| **Suds suppressor** (such as silicone and/or fatty acid) | from 0wt% to 0.1wt% |
| **Perfume** (such as perfume microcapsule, spray-on perfume, starch encapsulated perfume accords, perfume loaded zeolite, and any combination thereof) | from 0.1wt% to 1wt% |
| **Aesthetics** (such as coloured soap rings and/or coloured speckles/noodles) | from 0wt% to 1wt% |
| **Miscellaneous** | Balance |

**Laundry detergent pouch compositions:**

| Ingredients | A (wt%) | B (wt%) |
|---|---|---|
| $C_{14-15}$ alkyl poly ethoxylate (8) | 12 | 12 |

(continued)

| Ingredients | A (wt%) | B (wt%) |
|---|---|---|
| $C_{12-14}$ alkyl poly ethoxylate (7) | 1 | 1 |
| $C_{12-14}$ alkyl poly ethoxylate (3) sulfate Mono EthanolAmine salt | 8.4 | 8.4 |
| Linear Alkylbenzene sulfonic acid | 15 | 15 |
| Citric Acid | 0.6 | 0.6 |
| $C_{12-18}$ Fatty Acid | 15 | 15 |
| Enzymes | 1.5 | 1.5 |
| PEI 600 EO20 | 4 | 4 |
| Diethylene triamine penta methylene phosphonic acid or HEDP | 1.3 | 1.3 |
| Fluorescent brightener | 0.2 | 0.2 |
| Hydrogenated Castor Oil | 0.2 | 0.2 |
| 1, 2 propanediol | 16 | 16 |
| Glycerol | 6.2 | 6.2 |
| Sodium hydroxide | - | - |
| Mono Ethanol Amine | 7.9 | 7.9 |
| Dye | Present | Present |
| PDMS | - | - |
| Potassium sulphite | 0.2 | 0.2 |
| Benefit delivery composition (Sample 2) | 7.7 | 23.1 |
| Balance on Water, Surfactant and Fatty Acid | Up to 100% | Up to 100% |

**Hair Conditioner composition:**

| Components | (wt%) |
|---|---|
| Stearamidopropyldimethylamine (SAPDMA), C18 | 0.60 - 0.8 |
| DTDMAC, C18 (Quaternium-18) | 0.45 - 0.6 |
| Citric Acid (anhydrous) | 0.10 - 0.25 |
| Cetyl alcohol | 0.80 - 1.0 |
| Stearyl alcohol | 0.54 - 1.0 |
| Deionized Water | Balance |
| **Polymers** | |
| Hydroxyethylcellulose (HEC) | 0.15 - 0.50 |
| PEG-2M (Polyox WAR N-10) | 0.30 - 0.60 |
| **Others** | |
| Benefit delivery composition (sample 2) | 2.0 - 3.0 |
| Preservatives | 0.40 - 0.60 |

**Shampoo Composition:**

| | I (wt%) | II (wt%) |
|---|---|---|
| Ingredient | | |
| Water | balance | Balance |
| Polyquaternium 76 [1] | 2.50 | 2.50 |
| Guar, Hydroxylpropyl Trimonium Chloride [2] | -- | -- |
| Polyquaterium 6 [3] | - | - |
| Sodium Laureth Sulfate (SLE3S) [4] | 21.43 | 21.43 |
| Sodium Lauryl Sulfate (SLS) [5] | 20.69 | 20.69 |
| Cocoamidopropyl Betaine [6] | 3.33 | 3.33 |
| Cocoamide MEA [7] | 1.0 | 1.0 |
| Ethylene Glycol Distearate [8] | 1.50 | 1.50 |
| Sodium Chloride [9] | 0.25 | 0.25 |
| Benefit delivery composition (sample 2) | 0.75 | 1.5 |
| Preservatives, pH adjusters | Up to 1% | Up to 1% |
| 1 Mirapol AT-1, Copolymer of Acrylamide(AM) and TRIQUAT, MW=1,000,000; CD= 1.6 meq./gram; 10% active ; Supplier Rhodia<br>2 Jaguar C500, MW - 500,000, CD=0.7, supplier Rhodia<br>3 Mirapol 100S, 31.5% active, supplier Rhodia<br>4 Sodium Laureth Sulfate, 28% active, supplier: P&G<br>5 Sodium Lauryl Sulfate, 29% active supplier: P&<br>6 Tegobetaine F-B, 30% active supplier: Goldschmidt Chemicals<br>7 Monamid CMA, 85% active , supplier Goldschmidt Chemical<br>8 Ethylene Glycol Distearate, EGDS Pure, supplier Goldschmidt Chemical<br>9 Sodium Chloride USP (food grade), supplier Morton; note that salt is an adjustable ingredient, higher or lower levels may be added to achieve target viscosity. | | |

**Skin Lotion composition:**

| **PHASE A** | I (wt%) | II (wt%) |
|---|---|---|
| Polyethylene wax [1] | 3.54 | 3.54 |
| DC-2503 Cosmetic Wax[2] | 7.08 | 7.08 |
| TiO2 Coated Mica | 1.00 | 1.00 |
| Fragrance Particles | 1.00 | 1.00 |
| **PHASE B** | | |
| Glycerin | 10.00 | 10.00 |
| Dexpanthenol | 0.50 | 0.50 |
| Pentylene Glycol | 3.00 | 3.00 |
| Hexamidine Diisethionate [3] | 0.10 | 0.10 |
| Niacinamide [4] | 5.00 | 5.00 |
| Methy Iparaben | 0.20 | 0.20 |
| Ethylparaben | 0.05 | 0.05 |
| Sodium Citrate | 0.20 | 0.20 |

(continued)

| PHASE B | | |
|---|---|---|
| Citric Acid | 0.03 | 0.03 |
| Sodium Benzoate | 0.05 | 0.05 |
| Sodium Chloride | 0.50 | 0.50 |
| FD&C Red #40 (1%) | 0.05 | 0.05 |
| Benefit delivery composition (sample 2) | 59.00 | 29.5 |
| Water | Up to to 100% | Up to to 100% |
| **Hardness at 21 °C (g)** | 33.3 | 15.4 |
| 1. Jeenate™ 3H polyethylene wax from Jeen™<br>2. Stearyl Dimethicone. Available from Dow Corning.<br>3. Hexamidine diisethionate, available from Laboratoires Serobiologiques.<br>4. Additionally or alternatively, the composition may comprise one or more other skin care actives, their salts and derivatives, as disclosed herein, in amounts also disclosed herein as would be deemed suitable by one of skill in the art. | | |

**Body Wash composition:**

| **Base Surfactant Phase Composition** | **Composition A (wt%)** | **Composition B (wt%)** |
|---|---|---|
| Sodium Trideceth Sulfate (sulfated from Trideceth-2, Stepan) | 10.3% | 10.3% |
| Cocamidopropyl Betaine | 3.08% | 3.08% |
| Trideceth-3 | 1.64% | 1.64% |
| Sodium Chloride | 4.75% | 4.75% |
| Guar Hydroxypropyltrimonium Chlroide (N-Hance CG-17 from Aqualon) | 0.53% | 0.53% |
| Xanthan Gum (Keltrol 1000 from CP Kelco) | 0.37% | 0.37% |
| Acrylates/C10-30 Alkylacrylate Cross Polymer (Aqupec SER-300C from Sumitomo) | 0.033% | 0.033% |
| Methyl chloro isothiazolinone and methyl isothiazolinone (Kathon CG, Rohm & Haas) | 0.0007% | 0.0007% |
| EDTA (Dissolvine NA 2x) | 0.15% | 0.15% |
| Sodium Benzoate | 0.34% | 0.34% |
| Citric Acid, titrate | pH=5.7 | pH=5.7 |
| Benefit delivery composition (sample 2) | 0.75 | 1.5 |
| Balanced on Surfactant, Water and Minors | balance | Balance |

**Shave Gel composition:**

| **Finished Product Material Chemical Name** | **wt%** |
|---|---|
| Stearic Acid | 4.55 |
| Triethanolamine | 3.7 |
| Lanolin | 5 |
| Glycerin | 2 |

(continued)

| Finished Product Material Chemical Name | wt% |
|---|---|
| Polyoxyethylene Sorbitan Monostearate | 6 |
| Benefit delivery composition (sample 2) | 50 |
| Water | balance |

**Dentifrice composition:**

| Finished Product Material Chemical Name | I (wt%) | II (wt%) |
|---|---|---|
| SASS (27.9% Soln) | 7.5 | 7.5 |
| Sorbitol (70% soln) | 40.5 | 40.5 |
| Cetyl alcohols | 0.175 | 0.175 |
| Stearyl alcohols | 2.0 | 2.0 |
| Benefit delivery composition (sample 2) | 25.0 | 25.0 |
| Stannous of SnCl2 | | 1000ppm |
| Zinc of Zinc Citrate | | 2500ppm |
| Filler | Balance | Balance |

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm". The citation of any document is not an admission that it is prior art with respect to any invention disclosed or claimed herein or that it alone, or in any combination with any other reference or references, teaches, suggests or discloses any such invention. Further, to the extent that any meaning or definition of a term in this document conflicts with any meaning or definition of the same term in a document incorporated, the meaning or definition assigned to that term in this document shall govern.

While particular embodiments of the present invention have been illustrated and described, it would be obvious to those skilled in the art that various other changes and modifications can be made without departing from the scope of the invention. It is therefore intended to cover in the appended claims all such changes and modifications that are within the scope of this invention.

## Claims

**1.** A process for making a consumer goods product comprising a benefit delivery composition, wherein the process comprises the steps of:

(a) contacting a surfactant and a fatty amphiphile to form a lamellar phase composition;
(b) contacting the lamellar phase composition with silicone to form the benefit delivery composition,
(c) contacting the benefit delivery composition with at least three different consumer goods products ingredients to form a consumer goods product,

wherein the fatty amphiphile has a melting point of at least 40°C, wherein in step (a) the fatty amphiphile is at a temperature above its melting point when it is contacted with the surfactant, wherein the fatty amphiphile within the lamellar phase composition is subsequently cooled to a temperature below the melting point of the fatty amphiphile, wherein the benefit delivery composition comprises from 50wt% to less than 90wt% lamellar phase composition and from greater than 10wt% to 50wt% silicone,
and wherein the fatty amphiphile is selected from fatty acid, fatty alcohol and mixtures thereof, wherein step (b) is carried out under conditions of low shear, having a maximum tip speed of $2.5ms^{-1}$, preferably $2.0ms^{-1}$, or even $1.5ms^{-1}$, and at maximum shear rate of $500s^{-1}$

2. A process according to any preceding claim, wherein the weight ratio of lamellar phase composition to silicone present in the benefit delivery agent is in the range of from 1:1 to 8:1.

3. A process according to any preceding claim, wherein the step (b) is carried out under conditions of low shear having a maximum tip speed of 1.5ms-1.

4. A process according to any preceding claim, wherein the surfactant is selected from alkyl benzene sulphonate, alkyl ethoxylated sulphate and mixtures thereof.

5. A process according to any preceding claim, wherein the molar ratio of surfactant to fatty amphiphile is in the range of from 1:1 to 2.5:1.

6. A process according to any preceding claim, wherein:

(a) the surfactant is selected from $C_{10}$-$C_{13}$ alkyl benzene sulphonate, $C_{12}$-$C_{15}$ alkyl ethoxylated sulphate and mixtures thereof; and
(b) the fatty amphilie is selected from $C_8$-$C_{16}$ fatty acid, $C_8$-$C_{16}$ fatty alcohol and mixtures thereof.

7. A process according to any preceding claim, wherein the benefit delivery composition comprises from 20wt% to 50wt% silicone.

**Patentansprüche**

1. Verfahren zum Herstellen eines Konsumgüterprodukts, umfassend eine vorteilhafte Abgabezusammensetzung, wobei das Verfahren die Schritte umfasst:

(a) Inkontaktbringen eines Tensids und eines Fettamphiphils zur Bildung einer Zusammensetzung der lamellaren Phase;
(b) Inkontaktbringen der Zusammensetzung der lamellaren Phase mit Silikon, um die vorteilhafte Abgabezusammensetzung zu bilden,
(c) Inkontaktbringen der vorteilhaften Abgabezusammensetzung mit wenigstens drei verschiedenen Konsumgüterproduktbestandteilen zur Bildung eines Konsumgüterprodukts,

wobei das Fettamphiphil einen Schmelzpunkt von wenigstens 40 °C aufweist, wobei sich in Schritt (a) das Fettamphiphil bei einer Temperatur oberhalb seines Schmelzpunktes befindet, wenn es mit dem Tensid in Kontakt gebracht wird,
wobei das Fettamphiphil in der Zusammensetzung der lamellaren Phase anschließend auf eine Temperatur unterhalb des Schmelzpunktes des Fettamphiphils gekühlt wird, wobei die vorteilhafte Abgabezusammensetzung zu 50 Gew.-% bis weniger als 90 Gew.-% Zusammensetzung der lamellaren Phase und zu mehr als 10 Gew.-% bis 50 Gew.-% Silikon umfasst,
und wobei das Fettamphiphil ausgewählt ist aus Fettsäure, Fettalkohol und Mischungen davon, wobei Schritt (b) unter Bedingungen geringer Scherung, mit einer maximalen Spitze von 2,5 $ms^{-1}$, vorzugsweise 2,0 $ms^{-1}$, oder sogar 1,5 $ms^{-1}$ und einer maximalen Scherrate von 500 $s^{-1}$ durchgeführt wird.

2. Verfahren nach einem der vorstehenden Ansprüche, wobei das Gewichtsverhältnis zwischen der Zusammensetzung der lamellaren Phase zu Silikon in der vorteilhaften Abgabezusammensetzung im Bereich von 1:1 bis 8:1 liegt.

3. Verfahren nach einem der vorstehenden Ansprüche, wobei Schritt (b) unter Bedingungen geringer Scherung mit einer maximalen Spitzengeschwindigkeit von 1,5 ms-1 ausgeführt wird.

4. Verfahren nach einem der vorstehenden Ansprüche, wobei das Tensid ausgewählt ist aus der Gruppe bestehend aus Alkylbenzolsulfonat, alkylethoxyliertem Sulfat und Mischungen davon.

5. Verfahren nach einem der vorstehenden Ansprüche, wobei das Molverhältnis von Tensid zu Fettamphiphil im Bereich von 1:1 bis 2,5:1 liegt.

6. Verfahren nach einem der vorstehenden Ansprüche, wobei:

(a) das Tensid ausgewählt ist aus $C_{10}$-$C_{13}$-Alkylbenzolsulfonat, alkylethoxyliertem $C_{12}$-$C_{15}$-Sulfat und Mischungen davon; und
(b) das Fettamphiphil ausgewählt ist aus $C_8$-$C_{16}$-Fettsäure, $C_8$-$C_{16}$-Fettalkohol und Mischungen davon.

**7.** Verfahren nach einem der vorstehenden Ansprüche, wobei die vorteilhafte Abgabezusammensetzung zu 20 Gew.-% bis 50 Gew.-% Silikon umfasst.

## Revendications

**1.** Procédé de fabrication d'un produit de biens de consommation comprenant une composition de libération d'effet bénéfique, dans lequel le procédé comprend les étapes consistant à :

(a) mettre en contact un agent tensioactif et un amphiphile gras pour former une composition de phase lamellaire ;
(b) mettre en contact la composition de phase lamellaire avec de la silicone pour former la composition de libération d'effet bénéfique,
(c) mettre en contact la composition de libération d'effet bénéfique avec au moins trois ingrédients de produits de biens de consommation différents pour former un produit de biens de consommation,

dans lequel l'amphiphile gras a un point de fusion d'au moins 40 °C, dans lequel à l'étape (a) l'amphiphile gras est à une température supérieure à son point de fusion lorsqu'il est mis en contact avec l'agent tensioactif,
dans lequel l'amphiphile gras au sein de la composition de phase lamellaire est ultérieurement refroidi à une température inférieure au point de fusion de l'amphiphile gras, dans lequel la composition de libération d'effet bénéfique comprend de 50 % en poids jusqu'à moins de 90 % en poids de composition de phase lamellaire et de plus de 10 % en poids jusqu'à 50 % en poids de silicone,
et dans lequel l'amphiphile gras est choisi parmi un acide gras, un alcool gras et des mélanges de ceux-ci, dans lequel l'étape (b) est effectuée dans des conditions de faible cisaillement, ayant une vitesse périphérique maximale de 2,5 $ms^{-1}$, de préférence 2,0 $ms^{-1}$, ou même 1,5 $ms^{-1}$, et à un taux de cisaillement maximal de 500 $s^{-1}$.

**2.** Procédé selon l'une quelconque des revendications précédentes, dans lequel le rapport pondéral de la composition de phase lamellaire à la silicone présente dans l'agent de libération d'effet bénéfique est dans la plage allant de 1:1 à 8:1.

**3.** Procédé selon l'une quelconque des revendications précédentes, dans lequel l'étape (b) est effectuée dans des conditions de faible cisaillement ayant une vitesse périphérique maximale de 1,5 ms-1.

**4.** Procédé selon l'une quelconque des revendications précédentes, dans lequel l'agent tensioactif est choisi parmi un sulfonate d'alkylbenzène, un sulfate d'alkyle éthoxylé et des mélanges de ceux-ci.

**5.** Procédé selon l'une quelconque des revendications précédentes, dans lequel le rapport molaire de l'agent tensioactif à l'amphiphile gras est dans la plage allant de 1:1 à 2,5:1.

**6.** Procédé selon l'une quelconque des revendications précédentes, dans lequel :

(a) l'agent tensioactif est choisi parmi un sulfonate d'alkylbenzène en $C_{10}$ à $C_{13}$, un sulfate d'alkyle éthoxylé en $C_{12}$ à $C_{15}$ et leurs mélanges ; et
(b) l'amphile gras est choisi parmi un acide gras en $C_8$ à $C_{16}$, un alcool gras en $C_8$ à $C_{16}$ et leurs mélanges.

**7.** Procédé selon l'une quelconque des revendications précédentes, dans lequel la composition de libération d'effet bénéfique comprend de 20 % en poids à 50 % en poids de silicone.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 2006024256 A
- US 20120093757 A
- US 20030223952 A
- WO 200315736 A
- WO 200424114 A
- WO 2006012767 A
- WO 2008110590 A


### Non-patent literature cited in the description


- Theory of Self-Assembly of Hydrocarbon Amphiphiles into Micelles and Bilayers. J. Chem. Soc., Faraday Trans. 1976, vol. 2, 1525-1568
- **A. LEO.** Comprehensive Medicinal Chemistry. Pergamon Press, 1990, vol. 4, 295
- Comprehensive Evaluation of the Melting Points of Fatty Acids and Esters Determined by Differential Scanning Calorimetry. *J. Am. Oil Chem. Soc.,* 2009, vol. 86, 843-856A
- The HLB System. ICI Americas Inc, 1987